# EUROPÄISCHE PATENTSCHRIFT

(11) **EP 2 945 704 B1**
(45) Veröffentlichungstag und Bekanntmachung des Hinweises auf die Patenterteilung: **18.10.2017**
(21) Anmeldenummer: 14707313.4
(22) Anmeldetag: 16.01.2014
(51) Int. Cl.: A61Q 5/00, A61K 8/64, A61Q 19/00, A61K 8/97, A61K 8/19, A61K 8/20, A61K 8/65, A61K 8/11, A61K 8/23

(54) **KOPFHAUT-PFLEGEPRÄPARATION**
SCALP CARE PREPARATION
PREPARATION SOIGNANTE POUR LE CUIR CHEVELU

(30) Priorität: 16.01.2013 AT 500272013
(43) Veröffentlichungstag der Anmeldung: 25.11.2015
(73) Patentinhaber: Hairdreams Haarhandels GmbH, 8051 Graz (AT)
(72) Erfinder: OTT, Gerhard, 118455 Singapur (SG)
(74) Vertreter: Patentanwälte Barger, Piso & Partner
(86) Internationale Anmeldenummer: PCT/AT2014/050014
(87) Internationale Veröffentlichungsnummer: WO 2014/110613

(56) Entgegenhaltungen:
- WO-A1-02/060408
- DE-A1-102006 046 076
- KR-A- 20070 032 459
- Lonza: "Revitalising Hair and Scalp Styling Serum HC-13", , 1. Januar 2013 (2013-01-01), XP055113537, Gefunden im Internet: URL:http://www.innovadex.com/documents/707 86.pdf?bs=762&b=41455&st=20 [gefunden am 2014-04-10] in der Anmeldung erwähnt
- Anonymous: "skincaremember.com | Nutra Peptide Sensory Serum", , 1. Januar 2013 (2013-01-01), XP055113474, Gefunden im Internet: URL:https://skincaremember.com/hair2/nutra -peptide-sensory-serum/ [gefunden am 2014-04-10] in der Anmeldung erwähnt
- Anonymous: "Merman Minerals Hair Care", , 1. Januar 2012 (2012-01-01), XP055113458, Gefunden im Internet: URL:http://www.mermanminerals.com/haircare .html [gefunden am 2014-04-10]
- Anonymous: "GNPD - Scalp Energizer", , 1. Juli 2009 (2009-07-01), XP055113475, Gefunden im Internet: URL:http://www.gnpd.com/sinatra/recordpage /1140884/from_search/CHyyLHgh0T/ [gefunden am 2014-04-10] in der Anmeldung erwähnt

## Beschreibung

Die Erfindung bezieht sich auf eine Kopfhaut-Pflegepräparation, welche in einem gelförmigen Wasser-Alkohol-Gemisch Fenchelsamen-, Zahnstocherkraut- und Apfelfruchtextrakt sowie Hexapeptid-11 aufweist.

Derartige Präparate sind bereits in unzähligen Varianten - mit oder ohne
weiteren Ingredienzien - auf dem Weltmarkt. Beispielsweise seien genannt "Re-Vitalizing Hair & Scalp Styling Serum HC-13" von Arch Personal Care Products, "Nutra Peptide Sensory Serum" von Mermaid Minerals oder "Biojen 9 Hair & Scalp Rejuvenator" von Beauty Care Choices.

Fenchelsamen- und/oder Zahnstocherkrautextrakt fördern die Durchblutung der Kopfhaut durch verstärkte Mikrozirkulation im Bereich der Haarfollikel, Apfelfruchtextrakt und/oder Hexapeptid-11 zielen auf einen Hautzellenerneuerungseffekt ab und verbessern die Haarverankerung in der Kopfhaut, reduzieren also den Haarausfall. Hexapeptid-11 dürfte auch den biochemischen Umwandlungsvorgang vom Flaumhaar zum Terminalhaar positiv beeinflussen. Es ist in mehreren Sequenzformen bekannt, wie zB. der WO 2012/044745 A2 oder der US 2011/0052676 A1 entnehmbar.

Obzwar die Zusammensetzungen der bekannten Präparate mitunter stark voneinander abweichen, liegt der Anteil an Hexapeptid-11 und einem Fruchtextrakt, zB. Apfelfruchtextrakt, etwa im Bereich von 2 bis 6 Gew %, jener von Fenchelsamen- und/oder Zahnstocherkrautextrakt etwa in der Größenordnung von 1 Gew %. Die Wirkstoffkonzentrationen sind also relativ hoch.

Die Herstellerangaben bezüglich der empfohlenen Applikationen der Präparate variieren von einmal täglichem zu ein- bis zweimal wöchentlichem Einmassieren in die Kopfhaut. Die Präparate können allerdings dabei nur jene Aktivität entfalten, die der Oberflächentemperatur der Kopfhaut entspricht.

Aus der DE 10 2006 056 249 A1 ist ein Pflege- bzw. Reinigungsprodukt bekannt, das in einem Trägersubstrat (zB. Tuch oder Schwamm) eine "Benefit" - Zusammensetzung enthält, die wenigstens einen (zB. einen dermatologischen) Wirkstoff und eine bei Wasserkontakt Wärme freisetzende Substanz, zB. Kalziumchlorid oder Magnesiumsulfat, enthält, wobei letztere in Gelatinekapseln eingebettet sein kann, die während der Anwendung des Produktes infolge mechanischer Einwirkung zerstörbar sind.

Ziel der Erfindung ist eine Kopfhaut-Präparation mit im Vergleich zum Stand der Technik wesentlich geringerer, quasi homöopathischer, Wirkstoffkonzentration, deren Effektivität jedoch durch höhere, allerdings noch erträgliche, Temperaturen bei der Applikation mindestens gleichwertig, wenn nicht sogar gesteigert ist.

Dieses Ziel wird mit einer Kopfhaut-Pflegepräparation der eingangs genannten Art erfindungsgemäß dadurch erreicht, dass sie
0,030 bis 0,150 Gew % Fenchelsamenextrakt
0,030 bis 0,150 Gew % Zahnstocherkrautextrakt
0,005 bis 0,030 Gew % Apfelfruchtextrakt
0,001 bis 0,030 Gew % Hexapeptid-11
sowie 1,000 bis 5,000 Gew % eines in zerquetschbare Weichkapseln, beispielsweise aus Gelatine, eingebetteten thermoaktiven Wirkstoffs, beispielsweise Kalziumchlorid oder Magnesiumsulfat, enthält.

Durch Versuchsreihen konnte die Wirkstoffkonzentration gegenüber dem Stand der Technik erheblich verringert werden, und zwar ohne nennenswerten Aktivitätsverlust. Dabei hat sich herausgestellt, dass die jeweilige Sequenz von Hexapeptid-11 nicht ausschlaggebend ist. Ein Aktivitätsverlust - falls überhaupt gelegentlich vorhanden - wird aber jedenfalls durch die Zugabe des thermoaktiven Wirkstoffes nicht nur kompensiert, sondern sogar gesteigert. Dadurch, dass der thermoaktive Wirkstoff in an sich bekannter Weise zunächst in Weichkapseln eingebettet ist, die erst beim Einmassieren des Präparats zerquetscht werden, wird eine lokale Oberflächenerwärmung der Kopfhaut bewirkt, die die Aktivität aller Wirkstoffe steigert. Durch die Erwärmung und anschließende Abkühlung auf die übliche Oberflächentemperatur der Kopfhaut wird diese im Prinzip einer "Kneippkur" unterzogen, dh es wird die Stimulanz von Wärme und Kälte ausgenutzt.

Bevorzugterweise können der eben umrissenen erfindungsgemäßen Kopfhaut-Pflegepräparation noch 1,0 bis 5,0 Gew % Siliziumdioxid in Pelletform mit einer Größe von 500 bis 5000 nm beigemengt sein, um beim Einmassieren einen Peelingeffekt zu erzielen, dh abgestorbene Hautzellen zu entfernen bzw die oberste Hornschicht der Haut mechanisch abzuschaben. Die Siliziumdioxidpellets lösen sich dabei rückstandslos auf.

Weiters hat sich gezeigt, dass durch Zusatz von 5,0 bis 15,0 Gew % leicht alkalischen Thermalwassers die Kopfhautflora zusätzlich positiv stimuliert werden kann. Diese Thermalwässer haben einen pH-Wert von etwa 7,05 bis 7,50 und enthalten entweder zB gelöste Schwefelwasserstoffe in einer Konzentration von etwa 0,5 bis 25 mg/l und - neben weiteren Inhaltsstoffen wie dreiwertiges Eisen - zweiwertigen Schwefel in einer Menge von etwa 1,8 bis 18,5 mg/l. Die positive Wirkung von Thermalwasser bei der Behandlung und Heilung von dermatologischen Erkrankungen und seine keratolytischen Effekte sind hinlänglich bekannt.

Durch Zugabe von 0,10 bis 1,00 Gew % Eichenwurzelextrakt kann schließlich die Widerstandkraft der Haut weiter verbessert werden. Dieser wasserlösliche Pflanzenwirkstoff hat bekanntlich antientzündliche, antiallergische und heilungsfördernde Eigenschaften und wirkt überdies juckreizstillend.

Die Erfindung wird anhand einer beispielsweisen Rezeptur der ausschlaggebenden Bestandteile näher erläutert.

| | Gew % |
|---|---|
| Wasser (gereinigt, entsalzt) | 68,900 |
| SD Alkohol 40 | 10,000 |
| Fenchelsamenextrakt | 0,040 |
| Zahnstocherkrautextrakt | 0,050 |
| Apfelfruchtextrakt | 0,025 |
| Hexapeptid-11 | 0,015 |
| Gelatineweichkapseln gefüllt mit thermoaktivem Wirkstoff | |
| (zB. Alkali-Aluminiumsilikat) | 3,000 |
| Geliermittel | 0,750 |
| Verdickungsmittel | 0,500 |
| Konservierungsmittel | 0,750 |
| Weiters können div. Pflanzenextrakte | |
| (zB. Rohrzucker-, Zitronen-, Kamelienblätterextrakte uam) enthalten sein. | bis zu 0,200 Gew % |

In die fertige, gelförmige Kopfhaut-Pflegepräparation können noch kumulativ oder alternativ zugesetzt werden:

| | |
|---|---|
| Siliziumdioxidpellets (Teilchengröße 500 bis 5000 nm) | 1,0 bis 5,0 Gew % |
| Thermalwasser (pH-Wert 7,15, gelöste Schwefelwasserstoffe 24,9 mg/l, zweiwertiger Schwefel 18,5 mg/l) | 15,00 Gew % |
| Eichenwurzelextrakt | 0,10 Gew % |

Die vorstehende umrissene Präparation hat sich in den bislang durchgeführten Kurzeitstudien mindestens als gleichwertig mit den am Markt befindlichen Produkten erwiesen, obwohl sie wesentlich niedrigere Wirkstoffkonzentrationen aufweist. Im Gange befindliche Langzeitstudien deuten schon jetzt darauf hin, dass der Erwärmungseffekt beim Einmassieren des Präparates sogar noch eine Wirkungssteigerung nach sich ziehen dürfte.

## Patentansprüche

1. Kopfhaut-Pflegepräparation, welche in einem gelförmigen Wasser-Alkohol-Gemisch Fenchelsamen-, Zahnstocherkraut- und Apfelfruchtextrakt sowie Hexapeptid-11 aufweist, **dadurch gekennzeichnet, dass** sie
0,030 bis 0,150 Gew % Fenchelsamenextrakt
0,030 bis 0,150 Gew % Zahnstocherkrautextrakt
0,005 bis 0,030 Gew % Apfelfruchtextrakt
0,001 bis 0,030 Gew % Hexapeptid-11
sowie 1,000 bis 5,000 Gew % eines in zerquetschbare Weichkapseln, beispielsweise aus Gelatine, eingebetteten thermoaktiven Wirkstoffs, beispielsweise Kalziumchlorid oder Magnesiumsulfat, enthält.

2. Kopfhaut-Pflegepräparation nach Anspruch 1, **dadurch gekennzeichnet, dass** 1,0 bis 5,0 Gew % Siliziumdioxid in Pelletform mit einer Größe von 500 bis 5000 nm beigemengt sind.

3. Kopfhaut-Pflegepräparation nach Anspruch 1 oder 2, **dadurch gekennzeichnet, dass** 5,0 bis 15,0 Gew % leicht alkalisches Thermalwasser zugesetzt sind.

4. Kopfhaut-Pflegepräparation nach einem der Ansprüche 1 bis 3, **dadurch gekennzeichnet, dass** 0,10 bis 1,00 Gew % Eichenwurzelextrakt zugesetzt sind.

## Claims

1. Scalp-Care-Preparation comprising in a water-alcohol-mixture, being in gel form, Fennel seed, Toothpickweed and Apple fruit extract as well as Hexapeptide-11, **characterized in that** it contains
0,030 to 0,150 weight % Fennelseed extract
0,030 to 0,150 weight % Toothpickweed extract
0,005 to 0,030 weight % Apple fruit extract
0,001 to 0,030 weight % Hexapeptide-11
as well as 1,000 to 5,000 weight % of a thermoactive active substance, e.g. calcium chloride or magnesium sulfate, embedded in crushable soft capsules, e.g. from gelatine.

2. Scalp-Care-Preparation according to claim 1, **characterized in that** 1,0 to 5,0 weight % of Silicon dioxide are added in form of pellets having a size of 500 to 5000 nm.

3. Scalp-Care-Preparation according to claim 1 or 2, **characterized in that** 5,0 to 15,0 weight % of a slightly alkaline thermal water are added.

4. Scalp-Care-Preparation according to one of the claims 1 to 3, **characterized in that** 0,10 to 1,00 weight % of Oak root extract are added.

## Revendications

1. Préparation de soin pour le cuir chevelu, qui présente, dans un mélange eau-alcool sous forme de gel, un extrait de graines de fenouil, un extrait de khella et un extrait de pomus ainsi que de l'hexapeptide-11, **caractérisée en ce qu'**elle contient
0,030 à 0,150% en poids d'extrait de graines de fenouil
0,030 à 0,150% en poids d'extrait de khella
0,005 à 0,030% en poids d'extrait de pomus
0,001 à 0,030% en poids d'hexapeptide-11
ainsi que 1,000 à 5,000% en poids d'une substance active, thermoactive, par exemple du chlorure de calcium ou du sulfate de magnésium, incorporée dans des capsules molles pouvant être écrasées, par exemple en gélatine.

2. Préparation de soin pour le cuir chevelu selon la revendication 1, **caractérisée en ce que** 1,0 à 5,0% en poids de dioxyde de silicium sous forme de pellets d'une grosseur de 500 à 5000 nm y sont mélangés.

3. Préparation de soin pour le cuir chevelu selon la revendication 1 ou 2, **caractérisée en ce que** 5,0 à 15,0% en poids d'eau thermale légèrement alcaline y sont ajoutés.

4. Préparation de soin pour le cuir chevelu selon la revendication 1 à 3, **caractérisée en ce que** 0,10 à 1,00% en poids d'extrait de racine de chêne y est ajouté.
